(19) Europäisches Patentamt
European Patent Office
Office européen des brevets

(11) **EP 3 613 445 A1**

(12) **EUROPEAN PATENT APPLICATION**
published in accordance with Art. 153(4) EPC

(43) Date of publication:
**26.02.2020 Bulletin 2020/09**

(51) Int Cl.:
**A61L 27/30** (2006.01)   **A61F 2/30** (2006.01)
**A61L 15/18** (2006.01)   **A61L 17/00** (2006.01)
**A61L 17/14** (2006.01)   **A61L 27/40** (2006.01)
**A61L 29/10** (2006.01)   **A61L 29/12** (2006.01)
**A61L 31/08** (2006.01)   **A61L 31/12** (2006.01)
**A61L 33/02** (2006.01)   **C12M 1/00** (2006.01)

(21) Application number: 18788303.8

(22) Date of filing: 20.03.2018

(86) International application number:
**PCT/JP2018/011019**

(87) International publication number:
**WO 2018/193779 (25.10.2018 Gazette 2018/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 20.04.2017  JP 2017083711
30.06.2017  JP 2017128332

(71) Applicant: **Nikon Corporation**
**Tokyo 108-6290 (JP)**

(72) Inventors:
• **MORIYAMA, Masaki**
**Tokyo 108-6290 (JP)**
• **TAKAHASHI, Yuki**
**Tokyo 108-6290 (JP)**
• **TAKI, Yusuke**
**Tokyo 108-6290 (JP)**

(74) Representative: **Hoffmann Eitle**
**Patent- und Rechtsanwälte PartmbB**
**Arabellastraße 30**
**81925 München (DE)**

(54) **ARTICLE USED IN CONTACT WITH LIVING BODY OR BIOLOGICAL SAMPLE, MEDICAL INSTRUMENT, AND ARTIFICIAL JOINT**

(57)    An article used in contact with a living body or a biological sample is provided, the article including a first film and a second film, in which the first film includes an amorphous carbon film in which a proportion of the number of carbon atoms having an sp2-hybrid orbital to a total number of carbon atoms having an sp2-hybrid orbital and carbon atoms having an sp3-hybrid orbital is in a range of 23 to 43 atom% or a titanium-doped amorphous carbon film in which a proportion of the number of titanium atoms to the number of carbon atoms is in a range of 3 to 12 atom%, and the second film includes any one film selected from an amorphous carbon film in which a contact angle with pure water is 10° or less, a titanium-doped amorphous carbon film in which a proportion of the number of titanium atoms to the number of carbon atoms is less than 3 atom% or greater than 12 atom%, or a titanium-doped amorphous carbon film in which a contact angle with pure water is 10° or less.

FIG. 1

**Description**

TECHNICAL FIELD

The present invention relates to an article used in contact with a living body or a biological sample, a medical instrument, and an artificial joint. Priorities are claimed on Japanese Patent Application No. 2017-083711, filed on April 20, 2017, and Japanese Patent Application No. 2017-128332, filed on June 30, 2017, the content of which is incorporated herein by reference.

BACKGROUND ART

For example, the artificial joint disclosed in PTL 1 and medical instruments such as artificial bones, artificial dental roots, stents, catheters, pacemaker electrodes, pacemaker lead wires, contact lenses, intraocular lenses, and electric knives are used in contact with living bodies. Further, laboratory instruments such as cell culture dishes, test tubes, vials, and pipettes are also used in contact with biological samples such as cells, proteins, and body fluids derived from living bodies in some cases. These is a case where these articles have regions where blood compatibility, protein absorption properties, and cell adhesiveness are required to be excellent and regions where blood compatibility, protein absorption properties, and cell adhesiveness are required to be degraded conversely.

For example, in an artificial joint, the surface of the sliding portion of the joint needs to be as clean as possible without adhesion of proteins or cells thereto, and the connection portion with a living body needs to fully adhere to the living body. Further, for example, in a stent disposed in the blood vessels, the inner surface thereof which is constantly exposed to the blood needs to have high blood compatibility, and the outer surface thereof which is in contact with the blood vessels preferably has high cell adhesiveness.

Citation List

Patent Literature

[PTL 1] Japanese Unexamined Patent Application, First Publication No. 2014-104175

DISCLOSURE OF INVENTION

According to an embodiment, an article used in contact with a living body or a biological sample is provided, the article including: a first film and; a second film, in which the first film includes an amorphous carbon film in which a proportion of the number of carbon atoms having an $sp^2$-hybrid orbital to a total number of carbon atoms having an $sp^2$-hybrid orbital and carbon atoms having an $sp^3$-hybrid orbital is in a range of 23 to 43 atom% or a titanium-doped amorphous carbon film in which a proportion of the number of titanium atoms to the number of carbon atoms is in a range of 3 to 12 atom%, and the second film includes any one film selected from an amorphous carbon film in which a static contact angle with pure water is 10° or less, a titanium-doped amorphous carbon film in which a proportion of the number of titanium atoms to the number of carbon atoms is less than 3 atom% or greater than 12 atom%, or a titanium-doped amorphous carbon film in which a static contact angle with pure water is 10° or less.

According to an embodiment, the article is a medical instrument.

According to an embodiment, the article is an artificial joint.

According to an embodiment, a medical instrument is provided, which has at least a portion coated with an amorphous carbon film in which a proportion of the number of carbon atoms having an $sp^2$-hybrid orbital to a total number of carbon atoms having an $sp^2$-hybrid orbital and carbon atoms having an $sp^3$-hybrid orbital is in a range of 23 to 43 atom% or a titanium-doped amorphous carbon film in which a proportion of the number of titanium atoms to the number of carbon atoms is in a range of 3 to 12 atom%.

According to an embodiment, a medical instrument is provided, which has at least a portion coated with any one film selected from an amorphous carbon film in which a static contact angle with pure water is 10° or less, a titanium-doped amorphous carbon film in which a proportion of the number of titanium atoms to the number of carbon atoms is less than 3 atom% or greater than 12 atom%, and a titanium-doped amorphous carbon film in which a static contact angle with pure water is 10° or less.

BRIEF DESCRIPTION OF DRAWINGS

FIG. 1 is a schematic view describing a structure of an article according to an embodiment.

FIG. 2 is a schematic configuration view illustrating a filtered cathodic vacuum arc (FCVA) apparatus.

FIG. 3 is a graph showing results obtained by measuring the albumin adsorption amount in each sample of Experimental Example 2.

FIG. 4 is a graph showing results obtained by measuring the fibrinogen adsorption amount in each sample of Experimental Example 2.

FIG. 5 is a graph showing results obtained by measuring the cell adhesion rate in each sample of Experimental Example 3.

FIG. 6 is a graph showing results obtained by measuring the cell viability in Experimental Example 4.

FIG. 7 is a graph showing results obtained by measuring the hemolysis rate of the blood in contact with each sample in Experimental Example 5.

FIG. 8 is a graph showing results obtained by measuring the concentration of a thrombin-antithrombin complex (TAT) serving as an indicator of the blood coagulation, the concentration of $\beta$-thromboglobulin ($\beta$-TG) serving as an indicator of the platelet activity, and the concentration of C5a serving as an indicator of the inflammatory reaction in Experimental Example 5.

FIG. 9 shows representative electron microphotographs from the blood coagulation test in Experimental Example 5 on the graph showing the fibrinogen adsorption amount of each sample measured in Experimental Example 2.

FIG. 10 (a) to FIG. 10 (d) are schematic views illustrating a surface chemical structure of each sample film as determined by time-of-flight secondary ion mass spectrometry (TOF-SIMS) analysis.

FIG. 11 (a) and FIG. 11 (b) are schematic views illustrating an interaction between an amorphous carbon film and water molecules.

FIG. 12 (a) and FIG. 12 (b) are schematic views illustrating an interaction between a titanium-doped amorphous carbon film and water molecules.

FIG. 13 (a) and FIG. 13 (b) are schematic views each describing a structure of an article according to an embodiment.

BEST MODE FOR CARRYING OUT THE INVENTION

Hereinafter, embodiments of the present invention will be described in detail with reference to the accompanying drawing in some cases. In the drawings, the same or corresponding portions are denoted by the same or corresponding reference numerals, and the description thereof will not be provided. Further, the dimensional ratios in each drawing are exaggerated for description and do not necessarily match the actual dimensional ratios.

[Article used in contact with living body or biological sample]

An article according to the present embodiment is an article used in contact with a living body or a biological sample. The article includes a first film and a second film. The first film includes an amorphous carbon film in which a proportion of the number of carbon atoms having an $sp^2$-hybrid orbital to a total number of carbon atoms having an $sp^2$-hybrid orbital and carbon atoms having an $sp^3$-hybrid orbital is in a range of 23 to 43 atom% or a titanium-doped amorphous carbon film in which a proportion of the number of titanium atoms to the number of carbon atoms is in a range of 3 to 12 atom%, and the second film includes any one film selected from an amorphous carbon film in which a static contact angle with pure water is 10° or less, a titanium-doped amorphous carbon film in which a proportion of the number of titanium atoms to the number of carbon atoms is less than 3 atom% or greater than 12 atom%, or a titanium-doped amorphous carbon film in which a static contact angle with pure water is 10° or less.

Further, the amorphous carbon film in the present specification is a carbon film containing both carbon atoms having an $sp^2$-hybrid orbital and carbon atoms having an $sp^3$-hybrid orbital. A carbon film containing both carbon atoms described above is referred to as an amorphous carbon film regardless of the content of the carbon atoms having an $sp^2$-hybrid orbital and the carbon atoms having an $sp^3$-hybrid orbital. In the present specification, in a case where the amorphous carbon film contains titanium atoms, this amorphous carbon film is referred to as a titanium-doped amorphous carbon film.

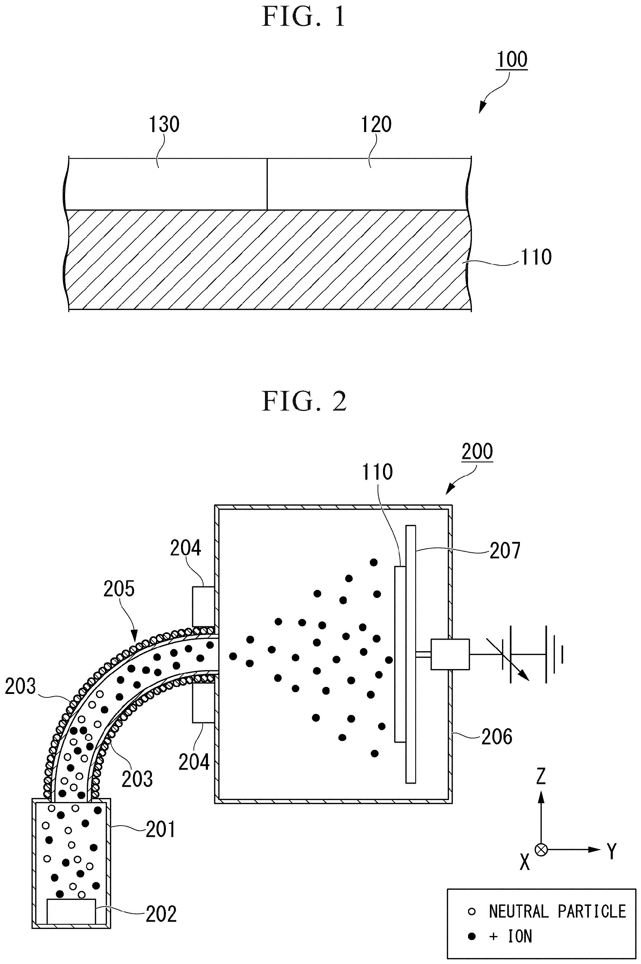

FIG. 1 is a schematic view describing the structure of the article according to the present embodiment. An article 100 is used in contact with a living body or a biological sample. The article 100 includes a base material (substrate) 110, and a first film 120 and a second film 130 which are laminated on the base material 110.

The article 100 may be a laboratory instrument such as a medical instrument, a cell culture dish, a test tube, a diagnostic chip, a chemical sensor, a biosensor, a vial, or a pipette.

The base material 110 is not particularly limited, and examples thereof which can be used include a resin, silicon, titanium, and stainless steel (particularly medical stainless steel).

(Blood compatibility)

As described below in examples, the present inventors clarified that the first film 120 tends to have high blood compatibility and the second film 130 tends to have low blood compatibility. In the present specification, the expression "the blood compatibility of a film is high" means that the degree of hemolysis or blood coagulation occurring in a case where the film is brought into contact with the blood is the same or less than that of a control material.

Here, as the control material, a material that is clearly known to have high blood compatibility in advance can be used, and examples thereof which may be used include a high-density polyethylene film of the related art and a blood bag. As described below in examples, the blood compatibility of a film can be measured by performing a hemolysis test or a blood coagulation test.

Further, the expression "the blood compatibility of a film is low" means that the degree of hemolysis or blood coagulation occurring in a case where the film is brought into contact with the blood is higher than that of a control material. Here, the control material is the same as described above.

As described below in examples, the present inventors clarified that a film with high blood compatibility tends to have degraded protein adsorption properties or cell adhesiveness.
Further, the present inventors clarified that a film with low blood compatibility tends to have excellent protein adsorption properties or cell adhesiveness.

(First film)

In the article 100, the first film 120 is a film having high blood compatibility. The first film 120 can be set as a film having degraded protein adsorption properties or cell adhesiveness. As described below in examples, the present inventors clarified that an amorphous carbon film in which the proportion of the number of carbon atoms having an $sp^2$-hybrid orbital to a total number of carbon atoms having an $sp^2$-hybrid orbital and carbon atoms having an $sp^3$-hybrid orbital is in a range of 23 to 43 atom% or a titanium-doped amorphous carbon film in which the proportion of the number of titanium atoms to the number of carbon atoms is in a range of 3 to 12 atom% has high blood compatibility. Therefore, a film having any of these films can be used as the first film.

<<Amorphous carbon>>

The amorphous carbon film can be formed on the base material 110 by using a physical vapor deposition method (PVD method) or a chemical vapor deposition method (CVD method). In a case where the amorphous carbon film is formed by using a PVD method, for example, a method such as an ion beam deposition method, an ion beam sputtering method, a magnetron sputtering method, a laser deposition method, a laser sputtering method, an arc ion plating method, or a filter cathodic vacuum arc (FCVA) method may be employed using a carbon raw material as a target. In a case where the amorphous carbon film is formed by using a CVD method, for example, a method such as a microwave plasma CVD method, a DC plasma CVD method, a high-frequency plasma CVD method, or a magnetic field plasma CVD method may be employed using hydrocarbon gas as a raw material.

Among these, an FCVA method is preferable since this method is a film-deposition method that enables a base material having a complicated shape to be uniformly coated with a high adhesive force at room temperature. The FCVA method is a film-deposition method of generating ionized particles by causing arc discharge from a raw material target and guiding only these particles to the substrate 110 to form a film.

FIG. 2 is a schematic configuration view illustrating an FCVA apparatus 200. The FCVA apparatus 200 is formed such that an arc plasma generation chamber 201 where a raw material target 202 is installed is connected to a film deposition chamber 206 through a spatial filter 205.

The film deposition chamber 206 includes a substrate holder 207 therein. The substrate holder 207 fixes the substrate 110, and the substrate 110 can be inclined in a θX direction or rotate in a θY direction by driving means (not illustrated). The spatial filter 205 is double-bended in a -X axis direction and a Y axis direction. An electromagnetic coil 203 is wound around the spatial filter 205, and an ion scan coil 204 is wound around a communication unit with a film deposition chamber 206.

An amorphous carbon film can be formed by using a carbon raw material such as a graphite target as a raw material target. Further, an amorphous carbon film doped with a metal can be formed by using a target such as a graphite sintered body containing a metal as a raw material target. For example, the titanium-doped amorphous carbon film can be formed by using TiC as a raw material target. Further, the doped metal is not limited to Ti, and Na, K, Ca, B, Mg, Cu, Sr, Ba, Zn, Hf, Al, Zr, Fe, Co, Ni, V, Cr, Mo, W, Mn, Re, Ag, Au, Pt, Nb, Ta, or an alloy of two or more of these metals can be used. Further, the material to be doped is not limited to a metal, and a semiconductor material such as Si or H, N, or F may be doped.

In order to form the amorphous carbon film or the titanium-doped amorphous carbon film by using the FCVA method, first, a DC voltage is applied to the target 202 in the arc plasma generation chamber 201 to cause arc discharge, and thus arc plasma is generated.

Neutral particles in the generated arc plasma, $C^+$ ions, $Ti^+$ ions, $Ti^{2+}$ ions, $Ti^{3+}$ ions, $Ti^{4+}$ ions, and other ions are transported to the spatial filter 205, and the neutral particles are trapped by the electromagnetic coil 203 in the process of passing through the spatial filter 205. Therefore, only the $C^+$ ions, $Ti^+$ ions, $Ti^{2+}$ ions, $Ti^{3+}$ ions, $Ti^{4+}$ ions, and other ions are introduced into the film deposition chamber 206.

At this time, the flight direction of the ion flow can be moved to an optional direction by the ion scan coil 204. A negative bias voltage is applied to the substrate 110 in the film deposition chamber 206. The $C^+$ ions, $Ti^+$ ions, $Ti^{2+}$ ions, $Ti^{3+}$ ions, $Ti^{4+}$ ions, and other ions which are ionized by the arc discharge are accelerated by the bias voltage and deposited as a dense film on the substrate 110.

The amorphous carbon film formed in the above-described manner is a solid film formed of carbon atoms, and the carbon atoms are largely divided into carbon atoms having an $sp^2$-hybrid orbital and carbon atoms having an $sp^3$-hybrid orbital.

In the FCVA method, the content of the carbon atoms having an $sp^2$-hybrid orbital and the carbon atoms having an $sp^3$-hybrid orbital in the amorphous carbon film and the titanium-doped amorphous carbon film can be controlled by adjusting the bias voltage during the film deposition.

For example, the proportion of the number of carbon atoms having an $sp^2$-hybrid orbital to a total number of carbon atoms having an $sp^2$-hybrid orbital and carbon atoms having an $sp^3$-hybrid orbital in the amorphous carbon film can be set to be in a range of 23 to 43 atom% by adjusting the bias voltage. Further, for example, the proportion of the number of carbon atoms having an $sp^2$-hybrid orbital to the total number of carbon atoms having an $sp^2$-hybrid orbital, carbon atoms having an $sp^3$-hybrid orbital, and titanium atoms in the titanium-doped amorphous carbon film can be set to 60 atom% or greater by adjusting the bias voltage. Further, in a case where the proportion of the number of carbon atoms having an $sp^2$-hybrid orbital to a total number of carbon atoms having an $sp^2$-hybrid orbital and carbon atoms having an $sp^3$-hybrid orbital in the amorphous carbon film is set to $\alpha$ (atom%), $\alpha$ is represented by Equation (1) shown below.

$$\alpha \text{ (atom\%)} = (\text{number of } sp^2\text{-C atoms})/\{(\text{number of } sp^2\text{-C atoms}) + (sp^3\text{-C atoms})\} \times 100 \ldots (1)$$

[In Equation (1), (number of $sp^2$-C atoms) represents the number of carbon atoms having an $sp^2$-hybrid orbital in the amorphous carbon film, and (number of $sp^3$-C atoms) represents the number of carbon atoms having an $sp^3$-hybrid orbital in the amorphous carbon film.]

Further, in a case where the proportion of the number of carbon atoms having an $sp^2$-hybrid orbital to the total number of carbon atoms having an $sp^2$-hybrid orbital, carbon atoms having an $sp^3$-hybrid orbital, and titanium atoms in the titanium-doped amorphous carbon film is set to $\beta$ (atom%), $\beta$ is represented by Equation (2) shown below.

$$\beta \text{ (atom\%)} = (\text{number of } sp^2\text{-C atoms})/\{(\text{number of } sp^2\text{-C atoms}) + (sp^3\text{-C atoms}) + (\text{number of Ti atoms}\} \times 100 \ldots (2)$$

[In Equation (2), (number of $sp^2$-C atoms) represents the number of carbon atoms having an $sp^2$-hybrid orbital in the amorphous carbon film, (number of $sp^3$-C atoms) represents the number of carbon atoms having an $sp^3$-hybrid orbital in the amorphous carbon film, and (number of Ti atoms) represents the number of Ti atoms in the amorphous carbon film.]

According to the FCVA method, only the $C^+$ ions, $Ti^+$ ions, $Ti^{2+}$ ions, $Ti^{3+}$ ions, $Ti^{4+}$ ions, and other ions, in which variation in flight energy is suppressed, are introduced into the film deposition chamber 206, and the ion bombardment energy of various ion particles to be incident on the substrate 110 can be controlled by controlling the bias voltage to be applied to the substrate 110. Therefore, uniform film deposition can be made in the substrate 110 having a complicated shape.

<<Amorphous carbon film in which proportion of the number of carbon atoms having an $sp^2$-hybrid orbital to a total number of carbon atoms having an $sp^2$-hybrid orbital and carbon atoms having an $sp^3$-hybrid orbital is in range of 23 to 43 atoni%>>

The amorphous carbon film is formed by the FCVA method using carbon raw materials as the raw material target, and an amorphous carbon film in which the proportion of the number of carbon atoms having an sp$^2$-hybrid orbital to a total number of carbon atoms having an sp$^2$-hybrid orbital and carbon atoms having an sp$^3$-hybrid orbital is in a range of 23 to 43 atom% can be produced by adjusting the bias voltage during the film deposition.

As described below in examples, the present inventors clarified that the amorphous carbon film in which the proportion of the number of carbon atoms having an sp$^2$-hybrid orbital to a total number of carbon atoms having an sp$^2$-hybrid orbital and carbon atoms having an sp$^3$-hybrid orbital is in a range of 23 to 43 atom% has higher blood compatibility than that of a blood bag of the related art.

<<Titanium-doped amorphous carbon film in which proportion of number of titanium atoms to number of carbon atoms is in range of 3 to 12 atom%>>

A titanium-doped amorphous carbon film can be formed by the FCVA method using TiC as the raw material target. Further, the proportion of the titanium atoms in the titanium-doped amorphous carbon film can be adjusted by changing the content of the titanium atoms in the raw material target. Further, in a case where the proportion of the number of titanium atoms to the number of carbon atoms is set to γ (atom%), γ is represented by Equation (3) shown below.

$$\gamma\ (\text{atom\%}) = (\text{number of Ti atoms})/\{(\text{number of sp}^2\text{-C atoms}) + (\text{number of}$$

$$\text{sp}^3\text{-C atoms})\} \times 100 \ \dots \ (3)$$

[In Equation (3), (number of Ti atoms) represents the number of Ti atoms in the amorphous carbon film, (number of sp$^2$-C atoms) represents the number of carbon atoms having an sp$^2$-hybrid orbital in the amorphous carbon film, and (number of sp$^3$-C atoms) represents the number of carbon atoms having an sp$^3$-hybrid orbital in the amorphous carbon film.]

As described below in examples, the present inventors clarified that the titanium-doped amorphous carbon film in which the proportion of the number of titanium atoms to the number of carbon atoms is in a range of 3 to 12 atom% exhibits blood compatibility which is higher than or the same as the blood compatibility of a blood bag of the related art.

The proportion of the number of titanium atoms to the number of carbon atoms in the titanium-doped amorphous carbon film may be greater than 9 atom% and 12 atom% or less.

(Second film)

In the article 100, the second film 130 is a film having low blood compatibility. The second film 130 can be set as a film having excellent protein adsorption properties or cell adhesiveness. As described below in examples, the present inventors clarified that an amorphous carbon film in which the static contact angle with pure water is 10° or less, a titanium-doped amorphous carbon film in which the proportion of the number of titanium atoms to the number of carbon atoms is less than 3 atom% or greater than 12 atom%, and a titanium-doped amorphous carbon film in which the static contact angle with pure water is 10° or less have low blood compatibility. Therefore, any one of these films can be used as the second film.

<<Aniorphous carbon film in which static contact angle with pure water is 10° or less>>

In the amorphous carbon film formed by the FCVA method or the like, the static contact angle with pure water which is measured by a liquid droplet method is approximately 50° or greater regardless of the number of carbon atoms having an sp$^2$-hybrid orbital and the number of carbon atoms having an sp$^3$-hybrid orbital.

The static contact angle with pure water can be decreased by forming a functional group such as a hydroxyl group or a carboxyl group on the surface of this amorphous carbon film. The degree of a decrease in static contact angle with pure water is changed depending on the amount of the functional group to be formed and can be adjusted to, for example, 10° or less, 5° or less, or 4° or less.

The method of forming the functional group on the amorphous carbon film is not particularly limited, and the functional group can be formed by irradiating the amorphous carbon film with ultraviolet rays. The wavelength of ultraviolet rays and the irradiation amount of the ultraviolet rays can be appropriately adjusted. For example, the amorphous carbon film is irradiated with light having ultraviolet rays including a wavelength of 185 nm for approximately 20 minutes. At this time, for example, the light to be applied may have ultraviolet rays with a wavelength of 254 nm.

As described below in examples, the present inventors clarified that the amorphous carbon film in which the static contact angle with pure water is 10° or less is a film which has low blood compatibility, excellent protein adsorption properties, and high cell adhesiveness.

The static contact angle changes in some cases after a certain period of time even in a case where a functional group such as a hydroxyl group or a carboxyl group is formed on the surface of the amorphous carbon film, but the blood compatibility, the protein adsorption properties, and the cell adhesiveness of the film are maintained as long as the functional group is formed even in the case of the change in static contact angle, and the film functions as the second film. Therefore, the amorphous carbon film corresponds to the second film in a case where the static contact angle with pure water which is measured immediately after the treatment of forming the functional group on the surface of the amorphous carbon film, that is, the treatment of decreasing the static contact angle is 10° or less. Here, the term "immediately after" indicates within two minutes after the treatment.

Further, the second film can be defined as an amorphous carbon film in which the amount of the hydroxyl group and the carboxyl group is optionally larger than that of an amorphous carbon film serving as the first film.

<<Titanium-doped amorphous carbon film in which proportion of number of titanium atoms to number of carbon atoms is less than 3 atom% and greater than 12 atom%>>

As described above, a titanium-doped amorphous carbon film can be formed by the FCVA method using TiC as the raw material target. Further, the content of the titanium atoms in the titanium-doped amorphous carbon film can be adjusted by changing the content of the titanium atoms in the raw material target.

As described below in example, the present inventors clarified that the titanium-doped amorphous carbon film in which the proportion of the number of titanium atoms to the number of carbon atoms is less than 3 atom% and greater than 12 atom% is a film which has low blood compatibility, excellent protein adsorption properties, and high cell adhesiveness.

<<Titanium-doped amorphous carbon film in which static contact angle with pure water is 10° or less>>

In the titanium-doped amorphous carbon film formed by the FCVA method or the like, the static contact angle with pure water which is measured by a liquid droplet method is approximately 60° or greater in a range where the proportion of the number of titanium atoms to the number of carbon atoms is 2 atom% or greater.

The static contact angle with pure water can be decreased by forming a functional group such as a hydroxyl group or a carboxyl group on the surface of this titanium-doped amorphous carbon film. The degree of a decrease in static contact angle with pure water is changed depending on the amount of the functional group to be formed and can be adjusted to, for example, 10° or less, 5° or less, or 4° or less.

The method of forming the functional group on the titanium-doped amorphous carbon film is not particularly limited, and the functional group can be formed by irradiating the titanium-doped amorphous carbon film with ultraviolet rays. The wavelength of ultraviolet rays and the irradiation amount of the ultraviolet rays can be appropriately adjusted. For example, the amorphous carbon film is irradiated with light having ultraviolet rays including a wavelength of 185 nm for approximately 20 minutes.

As described below in examples, the present inventors clarified that the titanium-doped amorphous carbon film in which the static contact angle with pure water is 10° or less is a film which has low blood compatibility, excellent protein adsorption properties, and high cell adhesiveness.

The static contact angle changes in some cases after a certain period of time even in a case where a functional group such as a hydroxyl group or a carboxyl group is formed on the surface of the titanium-doped amorphous carbon film, but the blood compatibility, the protein adsorption properties, and the cell adhesiveness of the film are maintained as long as the functional group is formed even in the case of the change in static contact angle, and the film functions as the second film. Therefore, the titanium-doped amorphous carbon film corresponds to the second film in a case where the static contact angle with pure water which is measured immediately after the treatment of forming the functional group on the surface of the titanium-doped amorphous carbon film, that is, the treatment of decreasing the static contact angle is 10° or less. Here, the term "immediately after" indicates within two minutes after the treatment. Further, the second film can be defined as a titanium-doped amorphous carbon film in which the amount of the hydroxyl group and the carboxyl group is optionally larger than that of the titanium-doped amorphous carbon film serving as the first film.

The article 100 according to the present embodiment may include both the above-described first film 120 and the above-described second film 130. The first film 120 and the second film 130 can be optionally disposed depending on the purpose of use of the article. Further, the first film and the second film may be disposed such that portions thereof overlap each other. The first film 120 and the second film 130 may be disposed so as to cover the entire surface of the article 100 or may be disposed on only a part of the surface thereof.

Further, the article 100 may be formed by combining independent components or may be integrally molded.

In a case where the article 100 may be formed by combining independent components, the first film or the second film is formed on the surface of each component depending on the purpose of use of the article 100. Thereafter, the article 100 can be produced by combining respective components. In this manner, the article 100 partially having the first film or the second film can be produced.

In a case where the article 100 is integrally molded, the first film or the second film can be formed at a desired position of the article 100 by using the FCVA method or the like.

In this case, for example, at the time of forming the first film, the first film can be formed at a desired position by covering a position where the first film has not been formed with a mask.

Next, the article 100 partially having the first film or the second film can be produced using a method of covering a position where the second film has not been formed with a mask to form the second film and peeling the mask off from the position after the formation of the second film.

(Intermediate water)

Typically, in a polymer material in water, it has been said that nonfreezing water that strongly interacts with the polymer material and does not freeze even at a low temperature and free water that is not affected by the polymer material are present at the interface between the water and the polymer material, and intermediate water is present between the free water and the nonfreezing water.

For example, water can be classified into free water, intermediate water, and nonfreezing water based on the relaxation time for water molecules excited by the nuclear magnetic resonance to return to the original stable state. It has been said that the relaxation time of water molecules of free water is in a range of $10^{-12}$ to $10^{-11}$ seconds, the relaxation time of water molecules of intermediate water is in a range of $10^{-10}$ to $10^{-9}$ seconds, and the relaxation time of the motion of water molecules of nonfreezing water is in a range of $10^{-8}$ to $10^{-6}$. In other words, the free water has the highest mobility of water molecules, followed by the intermediate water and the nonfreezing water.

The nonfreezing water does not melt because the nonfreezing water does not freeze at a low temperature such as -100°C, the intermediate water melts at a temperature of -80°C or higher and lower than 0°C (in other words, the intermediate water has a melting point of -80°C or higher and lower than 0°C), and the free water melts at 0°C. Therefore, the presence or absence of intermediate water can be examined by allowing the surface water of the film to freeze at an extremely low temperature, performing thermal analysis (DSC) while heating the surface water, and detecting a change in the quantity of heat accompanied by the melting of water.

As described below in examples, the first film has high blood compatibility, degraded protein adsorption properties, and low cell adhesiveness. Further, the second film has low blood compatibility, excellent protein adsorption properties, and high cell adhesiveness. In this regard, the present inventors speculated that the state of intermediate water in the film surface and the presence or absence of intermediate water affect the blood compatibility and the like.

<<Amorphous carbon film>>

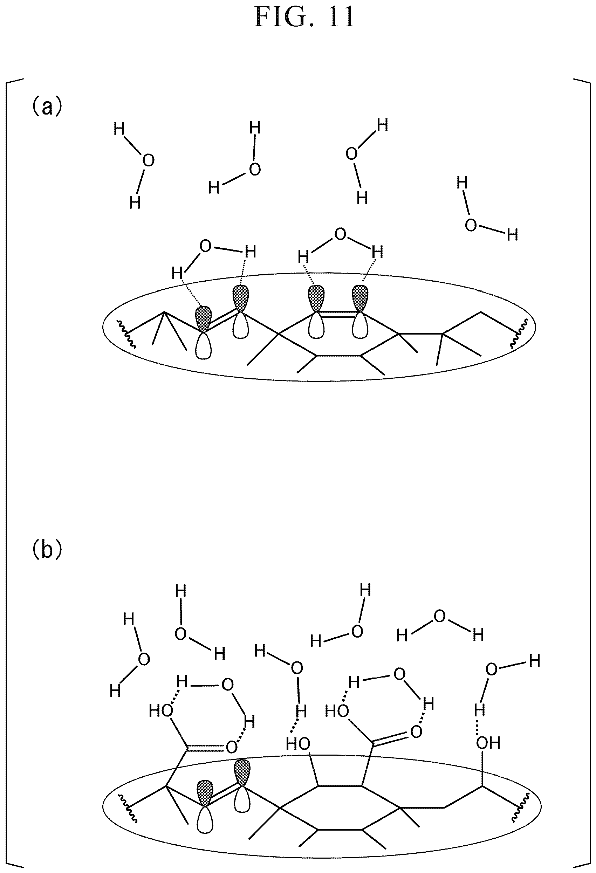

FIG. 11 (a) and FIG. 11 (b) are schematic views illustrating an interaction between an amorphous carbon film and water molecules.

As illustrated in FIG. 11 (a), an appropriate amount of π electrons at appropriate intervals are present in the amorphous carbon film. Accordingly, the π electrons interact with the water molecules as appropriate so that intermediate water is formed. In other words, intermediate water is present in the surface of the amorphous carbon film.

It is considered that the amorphous carbon film has high blood compatibility, degraded protein adsorption properties, and low cell adhesiveness because the amorphous carbon film has intermediate water. Particularly, the amorphous carbon film in which the proportion of the number of carbon atoms having an $sp^2$-hybrid orbital to a total number of carbon atoms having an $sp^2$-hybrid orbital and carbon atoms having an $sp^3$-hybrid orbital is in a range of 23 to 43 atom% has an appropriate amount of intermediate water and thus high blood compatibility is exhibited.

FIG. 11 (b) is a schematic view illustrating the interaction between water molecules and the amorphous carbon film having a functional group formed on the surface thereof. As illustrated in FIG. 11 (b), the water molecules and the functional group formed on the surface thereof strongly interact with each other in the amorphous carbon film having a functional group formed on the surface thereof. Therefore, nonfreezing water is present in the surface of the amorphous carbon film having a functional group formed on the surface thereof, and intermediate water almost does not exist. It is considered that in a case where nonfreezing water is present while the amount of intermediate water is small, denaturation and adsorption of proteins easily occur. Therefore, it is considered that the amorphous carbon film having a functional group formed on the surface thereof has low blood compatibility, excellent protein adsorption properties, and high cell adhesiveness. Further, it is considered that since the functional group directly interacts with proteins and adsorbs the proteins, the protein adsorption properties are excellent, the cell adhesiveness is high, and the blood compatibility is low.

The present inventors confirmed that the amorphous carbon film has intermediate water, and intermediate water disappears from the amorphous carbon film in a case where a functional group is formed on the surface thereof based on the DSC analysis.

<<Titanium-doped amorphous film>>

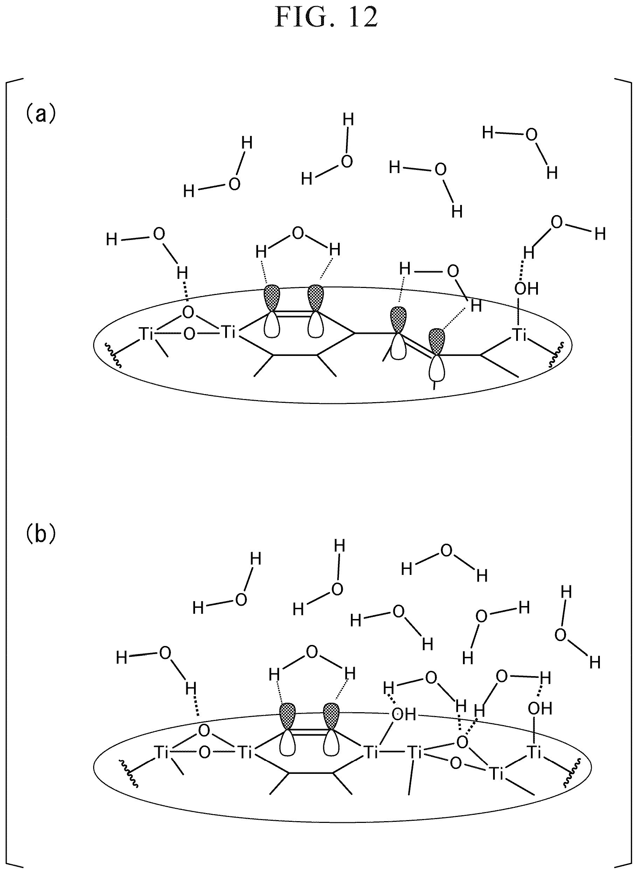

FIG. 12 (a) and FIG. 12 (b) are schematic views illustrating an interaction between a titanium-doped amorphous carbon film and water molecules.

As illustrated in FIG. 12 (a), it is considered that since an appropriate amount of titanium atoms are present in the titanium-doped amorphous carbon film in the form of TiC, TiO, TiOH, or the like, the water molecules are partially restrained, and the amount and the intervals of $\pi$ electrons become appropriate. Accordingly, it is considered that intermediate water is present in the surface of the titanium-doped amorphous carbon film.

It is considered that the titanium-doped amorphous carbon film has high blood compatibility, degraded protein adsorption properties, and low cell adhesiveness because the titanium-doped amorphous carbon film has intermediate water. Particularly, the titanium-doped amorphous carbon film in which the proportion of the number of titanium atoms to the number of carbon atoms is in a range of 3 to 12 atom% has an appropriate amount of intermediate water and thus high blood compatibility is exhibited.

Further, it is considered that in a case where the proportion of the number of titanium atoms to the number of carbon atoms in the titanium-doped amorphous carbon film is less than 3 atom%, the amount of water molecules to be restrained is not appropriate and intermediate water is not suitably formed. As a result, it is considered that the blood compatibility is low, the protein adsorption properties are excellent, and the cell adhesiveness is high.

Further, it is considered that in a case where the proportion of the number of titanium atoms to the number of carbon atoms is greater than 12 atom% in the titanium-doped amorphous carbon film, water molecules are easily restrained, the amount of the carbon skeleton decreases, and thus the amount of $\pi$ electrons becomes extremely small. As a result, it is considered that nonfreezing water is formed, the amount of intermediate water decreases, the blood compatibility becomes low, the protein adsorption properties become excellent, and the cell adhesiveness becomes high.

FIG. 12 (b) is a schematic view illustrating the interaction between water molecules and the titanium-doped amorphous carbon film having a functional group formed on the surface thereof. As illustrated in FIG. 12 (b), the water molecules and the functional group formed on the surface thereof strongly interact with each other in the titanium-doped amorphous carbon film having a functional group formed on the surface thereof. Therefore, nonfreezing water is present in the surface of the titanium-doped amorphous carbon film having a functional group formed on the surface thereof, and intermediate water almost does not exist. It is considered that in a case where nonfreezing water is present while the amount of intermediate water is small, denaturation and adsorption of proteins easily occur. Therefore, it is considered that the titanium-doped amorphous carbon film having a functional group formed on the surface thereof has low blood compatibility, excellent protein adsorption properties, and high cell adhesiveness. Further, it is considered that since the functional group directly interacts with proteins and adsorbs the proteins, the protein adsorption properties are excellent, the cell adhesiveness is high, and the blood compatibility is low.

The present inventors confirmed that the titanium-doped amorphous carbon film has intermediate water based on the DSC analysis.

(Division of first film or second film)

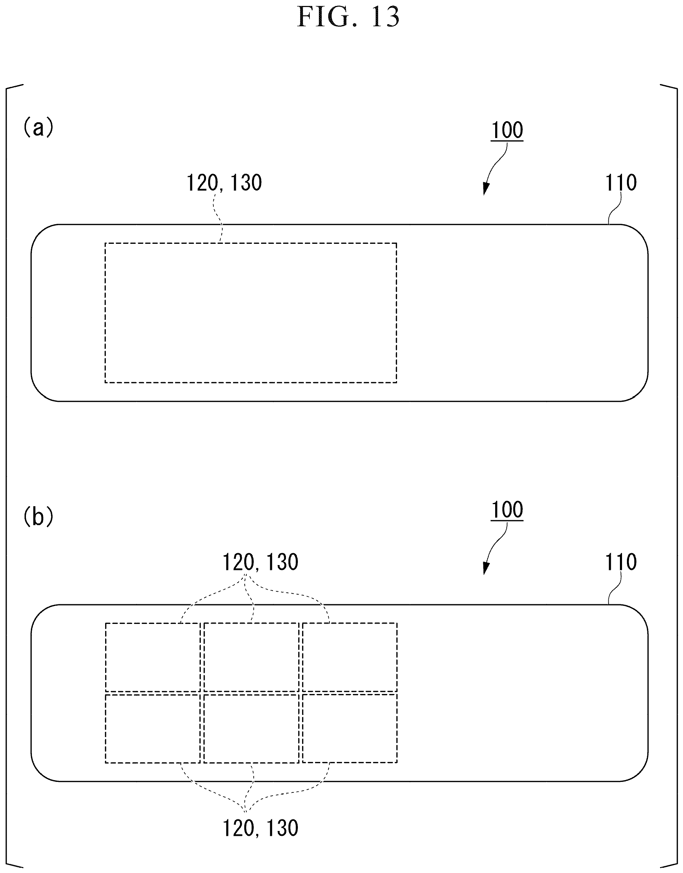

In the article 100, the first film 120 or the second film 130 may be divided into a plurality of parts. FIG. 13 (a) is a schematic view illustrating the article 100 in which division of the first film 120 or the second film 130 has not occurred. Further, FIG. 13 (b) is a schematic view illustrating the article 100 in which the first film 120 or the second film 130 is divided into a plurality of parts.

In the article 100, the base material 110 is deformed in some cases by being stretched or bended. Alternatively, the article 100 may be affected in some cases. Alternatively, the article 100 may be disposed at a sliding portion with another member. In such a case, the first film 120 or the second film 130 may be peeled off from the base material 110.

On the contrary, by dividing the first film 120 or the second film 130 into a plurality of parts, the influence caused from the deformation or impact of the base material is absorbed by the divided parts, and thus peeling of the first film 120 or the second film 130 can be suppressed.

The shape of the divided part of the first film 120 or the second film 130 is not particularly limited, and examples thereof include optional shapes such as a rectangle, a polygon, and a circle. Further, the shapes or sizes of respective divided parts of the first film 120 or the second film 130 may be the same as or different from each other.

As the method of dividing the first film 120 or the second film 130, a method of disposing a mesh-like mask on the base material 110 and forming the first film 120 or the second film 130 by using the above-described FCVA method may be exemplified. The mask is removed after the formation of the first film 120 or the second film 130. In this manner, the first film 120 or the second film 130 can be formed in a shape corresponding to the hollow portion of the mask after being divided at the mesh portion of the mask.

Alternatively, the division may be made by cutting the first film 120 or the second film 130 into a plurality of parts after formation of the first film 120 or the second film 130. The first film 120 or the second film 130 may be cut using a laser cutter or by etching.

[Medical instrument]

A medical instrument of the present embodiment is obtained by using the above-described article. In other words, the above-described article may be a medical instrument. Alternatively, the medical instrument of the present embodiment may be formed of the above-described article. Typically, the term "medical instrument" includes those which can be referred to as medical instruments, and specific examples thereof include artificial joints, artificial bones, artificial teeth, artificial dental roots, artificial hearts, artificial heart valves, artificial blood vessels, artificial anuses, artificial ureters, artificial pleura, prostheses, stents (including vascular stents and bronchial stents), guide wires, catheters, indwelling catheters, pacemaker electrodes, pacemaker lead wires, contact lenses, intraocular lenses, electric knives, injection needles, blood bags, blood-collection tubes, knives, endoscopes, filters such as blood filters, channels such as blood circuits, tubes such as blood-feeding tubes, forceps, artificial lungs, artificial heart-lung apparatuses, dialyzers, orthopedic instruments, artificial cochlea, artificial tympanic membranes, artificial larynx, cannulas, coils for cerebral artery treatment, artificial pancreas, acupuncture instruments, electrodes, sutures, wound covering materials, wound protecting materials, drain tubes, orthopedic implants, and pacemakers.

In the medical instrument of the present embodiment, it is preferable that the first film be provided in correspondence with a region of the article constituting the medical instrument in contact with the blood. In other words, it is preferable that the medical instrument of the present embodiment be formed such that the first film is present at a portion in contact with the blood in a case where the medical instrument is used in contact with a living body. In this case, since the first film has high blood compatibility, the medical instrument of the present embodiment suppresses the hemolysis or blood coagulation.

In the medical instrument of the present embodiment, it is preferable that the second film be provided in correspondence with a region of the article constituting the medical instrument in contact with a living body. In other words, it is preferable that the medical instrument of the present embodiment be formed such that the second film is present at a portion in contact with a living body in a case where the medical instrument is used in contact with a living body. In this case, since the second film has excellent protein adsorption properties and high cell adhesiveness, the medical instrument of the present embodiment tends to fully adhere to a living body.

In the medical instrument of the present embodiment, it is preferable that the first film or the second film be divided into a plurality of parts. For example, the first film or the second film disposed on the surface of the stent may be peeled off due to deformation or impact at the time of expansion of the stent in the blood vessels. In such a case, it is possible to suppress peeling of the first film or the second film by dividing the first film or the second film into a plurality of parts.

Examples of medical instruments which can be used by the first film and the second film include artificial joints, artificial bones, artificial teeth, artificial dental roots, artificial hearts, artificial heart valves, artificial blood vessels, artificial anuses, artificial ureters, artificial pleura, prostheses, stents (including vascular stents and bronchial stents), guide wires, catheters, indwelling catheters, pacemaker electrodes, pacemaker lead wires, contact lenses, intraocular lenses, electric knives, injection needles, blood bags, blood-collection tubes, channels such as blood circuits, tubes such as blood-feeding tubes, artificial lungs, artificial heart-lung apparatuses, dialyzers, orthopedic instruments, artificial cochlea, artificial tympanic membranes, artificial larynx, cannulas, coils for cerebral artery treatment, artificial pancreas, acupuncture instruments, electrodes, sutures, wound covering materials, wound protecting materials, drain tubes, orthopedic implants, and pacemakers.

The medical instrument of the present embodiment may be formed such that at least a portion thereof is coated with an amorphous carbon film in which the proportion of the number of carbon atoms having an $sp^2$-hybrid orbital to a total number of carbon atoms having an $sp^2$-hybrid orbital and carbon atoms having an $sp^3$-hybrid orbital is in a range of 23 to 43 atom% or a titanium-doped amorphous carbon film in which the proportion of the number of titanium atoms to the number of carbon atoms is in a range of 3 to 12 atom%. In other words, the medical instrument of the present embodiment may be formed such that at least a portion thereof is coated with the above-described first film. Further, the medical instrument of the present embodiment may not have the above-described second film.

Examples of medical instruments which can be used by the first film include artificial joints, artificial bones, artificial teeth, artificial dental roots, artificial hearts, artificial heart valves, artificial blood vessels, artificial anuses, artificial ureters, artificial pleura, prostheses, stents (including vascular stents and bronchial stents), guide wires, catheters, indwelling catheters, pacemaker electrodes, pacemaker lead wires, contact lenses, intraocular lenses, electric knives, injection needles, blood bags, blood-collection tubes, knives, endoscopes, filters such as blood filters, channels such as blood circuits, tubes such as blood-feeding tubes, forceps, artificial lungs, artificial heart-lung apparatuses, dialyzers, orthopedic instruments, artificial cochlea, artificial tympanic membranes, artificial larynx, cannulas, coils for cerebral artery treatment, artificial pancreas, acupuncture instruments, electrodes, sutures, wound covering materials, wound protecting materials, drain tubes, orthopedic implants, and pacemakers.

The medical instrument of the present embodiment may be formed such that at least a portion coated with any one film selected from an amorphous carbon film in which the static contact angle with pure water is 10° or less, a titanium-doped amorphous carbon film in which the proportion of the number of titanium atoms to the number of carbon atoms is less than 3 atom% or greater than 12 atom%, and a titanium-doped amorphous carbon film in which the static contact angle with pure water is 10° or less. In other words, the medical instrument of the present embodiment may be formed such that at least a portion thereof is coated with the above-described second film. Further, the medical instrument of the present embodiment may not have the above-described first film.

Examples of medical instruments which can be used by the second film include artificial joints, artificial bones, artificial teeth, artificial dental roots, artificial hearts, artificial heart valves, artificial blood vessels, artificial anuses, artificial ureters, artificial pleura, prostheses, stents (including vascular stents and bronchial stents), catheters, indwelling catheters, pacemaker electrodes, pacemaker lead wires, contact lenses, intraocular lenses, electric knives, channels such as blood circuits, artificial lungs, artificial heart-lung apparatuses, dialyzers, orthopedic instruments, artificial cochlea, artificial tympanic membranes, artificial larynx, cannulas, coils for cerebral artery treatment, artificial pancreas, acupuncture instruments, electrodes, sutures, wound covering materials, wound protecting materials, drain tubes, orthopedic implants, and pacemakers. As the second film, the titanium-doped amorphous carbon film containing titanium atoms at a high concentration is suitable for an electric knife.

[Artificial joint]

An artificial joint of the present embodiment may be obtained by using the above-described article. In other words, the above-described article may be an artificial joint.

Alternatively, the artificial joint of the present embodiment may be formed of the above-described article. In other words, the above-described article can be suitably used as the artificial joint. The artificial joint is not particularly limited, and examples thereof include an artificial shoulder joint, an artificial elbow joint, an artificial hip joint, and an artificial knee joint.

Typically, the artificial joint is formed of a plurality of members, and the sliding portion of each member needs to maintain as clean a state as possible without adhesion of proteins or cells thereto. In this case, it is preferable to form the first film which has degraded protein absorption properties and low cell adhesiveness. From the viewpoint of increasing the slidability, the sliding portion of each member is desired to be hydrophilic. In this case, it is preferable to form the second film. In other words, the first film and the second film can be appropriately selected and used in the artificial joint of the present embodiment depending on the required property.

Further, since the artificial joint is typically used in a living body, it is preferable that the second film having excellent protein adsorption properties and high cell adhesiveness be formed in a region in contact with the living body of the artificial joint. In other words, in the artificial joint of the present embodiment, it is preferable that the second film be provided in correspondence with the region in contact with the living body, among a plurality of members constituting the artificial joint.

In the artificial joint of the present embodiment, it is preferable that the first film or the second film be divided into a plurality of parts. For example, the first film or the second film disposed on the surface of the artificial joint may be peeled off due to deformation or impact particularly in the sliding portion and the like. In such a case, it is possible to suppress peeling of the first film or the second film by dividing the first film or the second film into a plurality of parts.

EXAMPLES

Hereinafter, the present embodiment will be described based on examples, but the present invention is not limited to the following examples.

[Experimental Example 1]

(Production of sample)

An amorphous carbon film or a titanium-doped amorphous carbon film was formed on a stainless steel (SUS316L) substrate or a PS (polystyrene) substrate by using an FCVA method to prepare a sample on which the film had been formed.

<<Deposition of amorphous carbon film>>

An amorphous carbon film was formed on a stainless steel substrate by using the FCVA method. The film deposition was performed by changing the bias voltage in four stages to prepare each sample. As a result of analysis performed on the film of each of the prepared sample surfaces according to the X-ray photoelectron spectroscopy (XPS), it became apparent that amorphous carbon films were obtained in which the proportions of the number of carbon atoms having an $sp^2$-hybrid orbital to a total number of carbon atoms having an $sp^2$-hybrid orbital and carbon atoms having an $sp^3$-hybrid orbital were respectively 22, 28, 37, and 44 atom%.

<<Deposition of titanium-doped amorphous carbon film>>

A titanium-doped amorphous carbon film was formed on a stainless steel substrate by using the FCVA method. The film deposition was performed using raw material targets with different contents of titanium atoms to prepare each sample. As a result of analysis performed on the film of each of the prepared sample surfaces by using XPS and Rutherford backscattering spectrometry (RBS) measurement, it became apparent that titanium-doped amorphous carbon films were obtained in which the proportions of the numbers of titanium atoms to the number of carbon atoms were respectively 2.0, 5.3, 12.4, and 28.2 atom%. Further, the proportions of the numbers of carbon atoms having an $sp^2$-hybrid orbital to the total numbers of carbon atoms having an $sp^2$-hybrid orbital, carbon atoms having an $sp^3$-hybrid orbital, and the titanium atoms in these titanium-doped amorphous carbon films were respectively in a range of 63 to 75 atom%.

<<Irradiation of film with ultraviolet rays>>

Each sample on which the amorphous carbon film or the titanium-doped amorphous carbon film had been formed was irradiated with light having ultraviolet rays including a wavelength of 185 nm for approximately 20 minutes, thereby preparing each surface-modified sample.
As described below, a hydroxyl group or a carboxyl group is formed on each film by the irradiation with ultraviolet rays. The static contact angle with pure water in each sample which had been irradiated with ultraviolet rays and each sample which had not been irradiated with ultraviolet rays was rapidly measured after the irradiation with ultraviolet rays.
The characteristics of each sample are collectively listed in Table 1. In Table 1, "performed" in the columns of "irradiation with UV" indicates that light having ultraviolet rays including a wavelength of 185 nm was applied, and "not performed" in the columns of "irradiation with UV" indicates that ultraviolet rays were not applied. The "$sp^2$-C (at%)" indicates the proportion (atom%) of the number of carbon atoms having an $sp^2$-hybrid orbital to the total number of carbon atoms having an $sp^2$-hybrid orbital and carbon atoms having an $sp^3$-hybrid orbital in a case of the amorphous carbon and indicates the proportion (atom%) of the number of carbon atoms having an $sp^2$-hybrid orbital to the total number of carbon atoms having an $sp^2$-hybrid orbital, carbon atoms having an $sp^3$-hybrid orbital, and titanium atoms in a case of the titanium-doped amorphous carbon. The "Ti/C (at%)" indicates the proportion (atom%) of the number of titanium atoms to the number of carbon atoms, and the "contact angle" indicates the static contact angle with pure water which was measured according to the liquid droplet method.

[Table 1]

| Samples | Explanation | Irradiation with UV | $sp^2$-C (at%) | Ti/C (at%) | Contact angle (°) |
|---|---|---|---|---|---|
| a | Only substrate | Not performed | - | - | 55.5 |
| b | Amorphous carbon | Not performed | 22 | - | 55.4 |
| c | Amorphous carbon | Not performed | 28 | - | 56.1 |
| d | Amorphous carbon | Not performed | 37 | - | 58.2 |
| e | Amorphous carbon | Not performed | 44 | - | 62.3 |
| f | Titanium-doped amorphous carbon | Not performed | 68 | 2.0 | 60.6 |
| g | Titanium-doped amorphous carbon | Not performed | 75 | 5.3 | 62.4 |
| h | Titanium-doped amorphous carbon | Not performed | 68 | 12.4 | 62.9 |
| i | Titanium-doped amorphous carbon | Not performed | 63 | 28.2 | 64.7 |
| j | Only substrate | Performed | - | - | 13.8 |
| k | Amorphous carbon | Performed | 22 | - | 4.4 |
| l | Amorphous carbon | Performed | 28 | - | 4.5 |
| m | Amorphous carbon | Performed | 37 | - | 4.3 |
| n | Amorphous carbon | Performed | 44 | - | 4.4 |

(continued)

| Samples | Explanation | Irradiation with UV | sp$^2$-C (at%) | Ti/C (at%) | Contact angle (°) |
|---|---|---|---|---|---|
| o | Titanium-doped amorphous carbon | Performed | 68 | 2.0 | 3.5 |
| p | Titanium-doped amorphous carbon | Performed | 75 | 5.3 | 3.5 |
| q | Titanium-doped amorphous carbon | Performed | 68 | 12.4 | 3.5 |
| r | Titanium-doped amorphous carbon | Performed | 63 | 28.2 | 3.4 |
| s | Only Ti | Not performed | - | 100.0 | - |

[Experimental Example 2]

(Examination of protein adsorption properties)

The protein adsorption properties of each film were evaluated using each sample prepared in Experimental Example 1. Albumin and fibrinogen were used as the proteins.

<<Albumin>>

First, each sample was immersed in an albumin solution at a concentration of 30 mg/mL and allowed to stand at 37°C for 24 ± 2 hours. Albumin was used by being dissolved in a phosphate buffer. Next, each sample was taken out from the albumin solution and washed with pure water. Next, each sample was immersed in a surfactant solution (obtained by dissolving 2 vol% Triton X-100 in a phosphate buffer) and shaken at 37°C for 30 minutes. Subsequently, the surfactant solution was recovered, and the eluted albumin was quantified.

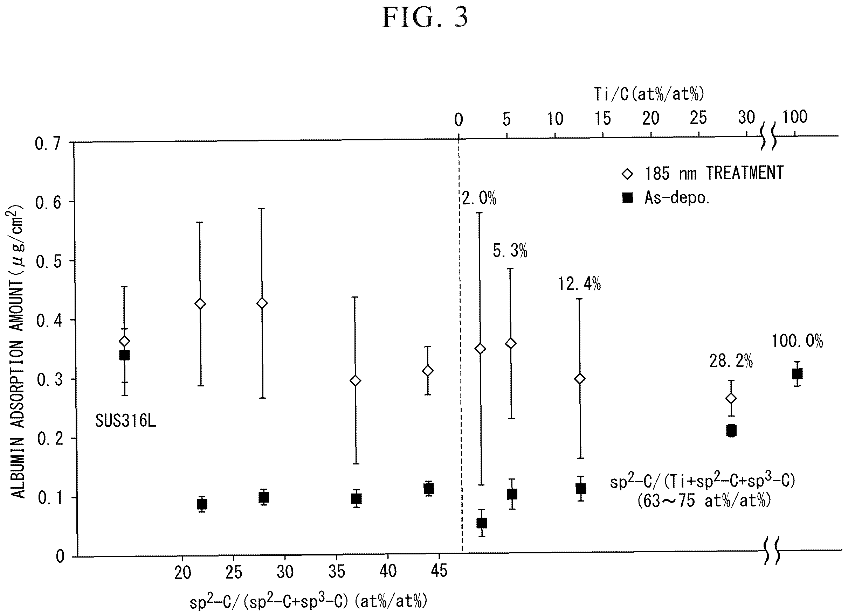

FIG. 3 is a graph showing the results obtained by measuring the albumin adsorption amount in each sample. In FIG. 3, the "185 nm treatment" indicates that a sample was irradiated with light having ultraviolet rays including a wavelength of 185 nm, the "As-depo." indicates that a sample was not irradiated with ultraviolet rays, "SUS316L" indicates that a sample was formed of only a substrate, and "at%" indicates atom%. Further, a sample in which Ti/C is 100 atom% results from a sample formed of only titanium which was measured for reference.

As a result, it was clarified that the proportion (atom%) of the number of carbon atoms having an sp$^2$-hybrid orbital to the total number of carbon atoms having an sp$^2$-hybrid orbital and carbon atoms having an sp$^3$-hybrid orbital in the amorphous carbon film (As-depo.) almost does not affect the albumin adsorption amount. On the contrary, it was clarified that the albumin adsorption amount was significantly increased in the amorphous carbon film (185 nm treatment) compared to a sample which was not irradiated with ultraviolet rays.

Further, there was a tendency that the albumin adsorption amount was increased in a case where the concentration of titanium was increased in the titanium-doped amorphous carbon film (As-depo.). Meanwhile, there was a tendency that the albumin adsorption amount was decreased along with an increase in the titanium concentration in the titanium-doped amorphous carbon film (185 nm treatment).

<<Fibrinogen>>

The protein adsorption properties of each film were evaluated using each sample prepared in Experimental Example 1 in the same manner as described above. Fibrinogen was used in place of albumin as the protein. The concentration of fibrinogen was set to 3 mg/mL.

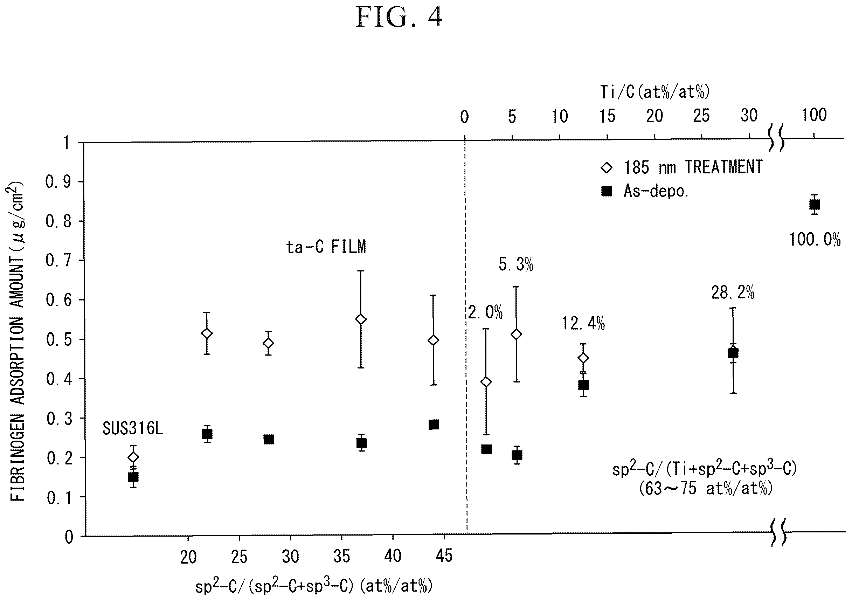

FIG. 4 is a graph showing the results obtained by measuring the fibrinogen adsorption amount in each sample. In FIG. 4, the "185 nm treatment" indicates that a sample was irradiated with light having ultraviolet rays including a wavelength of 185 nm, the "As-depo." indicates that a sample was not irradiated with ultraviolet rays, "SUS316L" indicates that a sample was formed of only a substrate, and "at%" indicates atom%. Further, a sample in which Ti/C is 100 atom% results from a sample formed of only titanium which was measured for reference.

As a result, it was found that the proportion (atom%) of the number of carbon atoms having an sp$^2$-hybrid orbital to the total number of carbon atoms having an sp$^2$-hybrid orbital and carbon atoms having an sp$^3$-hybrid orbital in the amorphous carbon film (As-depo.) was in a range of 23 to 43 atom%, and the fibrinogen adsorption amount was likely to be low. On the contrary, it was clarified that the fibrinogen adsorption amount was significantly increased in the amorphous carbon film (185 nm treatment) compared to a sample which was not irradiated with ultraviolet rays.

Further, in the titanium-doped amorphous carbon film (As-depo.), it was found that the proportion of the number of titanium atoms to the number of carbon atoms was 12 atom% or less and the fibrinogen adsorption amount was likely to be low, and the adsorption amount was likely to be minimized in a case where the proportion of the number of titanium atoms to the number of carbon atoms was 5 atom%. Meanwhile, the fibrinogen adsorption amount was not largely changed regardless of the titanium concentration in the titanium-doped amorphous carbon film (185 nm treatment). Further, it was found that the fibrinogen adsorption amount was likely to be increased compared to a sample which was not irradiated with ultraviolet rays.

[Experimental Example 3]

(Examination of cell adhesiveness)

The cell adhesiveness of each film was evaluated using each sample prepared in Experimental Example 1. As the cells, human umbilical vein endothelial cells (HUVEC) were used.

First, each sample was placed in a well of a cell culture well plate, HUVEC was seeded at $5 \times 10^4$ cells/well, and the cells were cultured in an environment of 37°C at $5\%CO_2$ for $24 \pm 2$ hours. Next, the cell adhesion rate was measured by fixing the cells in each well, staining the cell nuclei with fluorescence, and observing the cells using a fluorescence microscope.

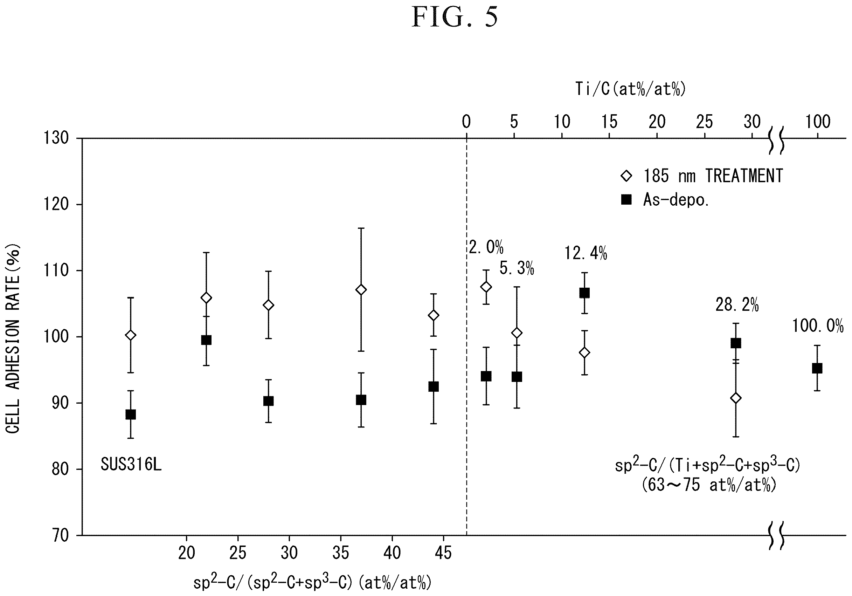

FIG. 5 is a graph showing the results obtained by measuring the cell adhesion rate to each sample. In FIG. 5, the "185 nm treatment" indicates that a sample was irradiated with light having ultraviolet rays including a wavelength of 185 nm, the "As-depo." indicates that a sample was not irradiated with ultraviolet rays, "SUS316L" indicates that a sample was formed of only a substrate, and "at%" indicates atom%. Further, a sample in which Ti/C is 100 atom% results from a sample formed of only titanium which was measured for reference. The cell adhesion rate indicates the proportion (%) obtained by setting the number of cells in a well of a cell culturing plate where a sample on which a film was formed was not placed to 100%.

As a result, in the amorphous carbon film (As-depo.), it was clarified that the proportion (atom%) of the number of carbon atoms having an sp$^2$-hybrid orbital to the total number of carbon atoms having an sp$^2$-hybrid orbital and carbon atoms having an sp$^3$-hybrid orbital was in a range of 23 to 43 atom% and the cell adhesion rate was likely to be low. On the contrary, it was clarified that the cell adhesion rate was significantly increased in the amorphous carbon film (185 nm treatment) compared to a sample which was not irradiated with ultraviolet rays.

Further, in the titanium-doped amorphous carbon film (As-depo.), it was clarified that the cell adhesion rate was maximized in a case where the proportion of the number of titanium atoms to the number of carbon atoms was 12.4 atom%. Meanwhile, there was a tendency that the cell adhesion rate was decreased along with an increase in the titanium concentration in the titanium-doped amorphous carbon film (185 nm treatment).

[Experimental Example 4]

(Toxicity test)

The toxicity with respect to living bodies or cells was examined using each sample prepared in the same manner as in Experimental Example 1. More specifically, a cytotoxicity test and an animal implantation test were performed.

<<Cytotoxicity test>>

Each sample was immersed in a culture medium and allowed to stand at 37°C for $24 \pm 2$ hours, thereby preparing an extract. Next, L929 cells as mouse fibroblasts were cultured in an environment of 37°C at $5\%CO_2$ for $24 \pm 2$ hours using this extract. Thereafter, the cell viability was measured by observing each cell.

As the samples, samples No. 1 to No. 5 listed in Table 2 were used. In Table 2, "performed" in the columns of "irradiation with UV" indicates that light having ultraviolet rays including a wavelength of 185 nm was applied, and "not performed" in the columns of "irradiation with UV" indicates that ultraviolet rays were not applied. The "sp$^2$-C (at%)" indicates the proportion (atom%) of the number of carbon atoms having an sp$^2$-hybrid orbital to the total number of carbon atoms having an sp$^2$-hybrid orbital and carbon atoms having an sp$^3$-hybrid orbital in a case of the amorphous carbon and indicates the proportion (atom%) of the number of carbon atoms having an sp$^2$-hybrid orbital to the total number of carbon atoms having an sp$^2$-hybrid orbital, carbon atoms having an sp$^3$-hybrid orbital, and titanium atoms in a case of the titanium-doped amorphous carbon. The "Ti/C (at%)" indicates the proportion (atom%) of the number of titanium atoms to the number of carbon atoms, and the "contact angle" indicates the static contact angle with pure water which was measured according to the liquid droplet method.

[Table 2]

| Samples | Explanation | Irradiation with UV | sp$^2$-C (at%) | Ti/C (at%) | Contact angle (°) |
|---|---|---|---|---|---|
| No.1 | Amorphous carbon | Not performed | 22 | - | 55.4 |
| No.2 | Amorphous carbon | Not performed | 44 | - | 62.3 |
| No.3 | Amorphous carbon | Performed | 22 | - | 4.4 |
| No.4 | Titanium-doped amorphous carbon | Not performed | 63 | 28.2 | 64.7 |
| No.5 | Only substrate | Not performed | - | - | 55.5 |

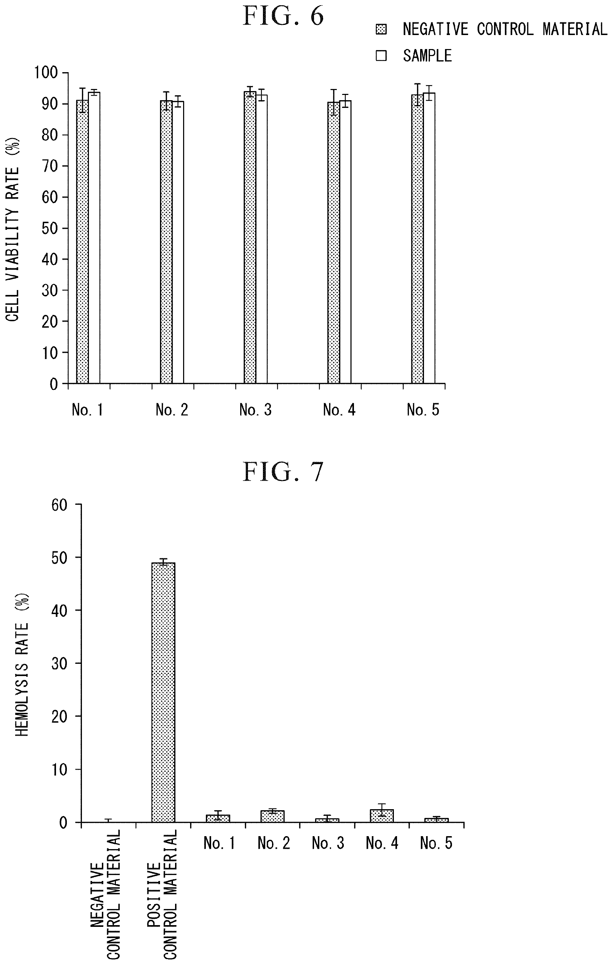

The cytotoxicity was not recognized as a result of observing each sample using a microscope. FIG. 6 is a graph showing the results obtained by measuring the cell viability. As the negative control material, the toxicity of a cell storage vial material was examined. In FIG. 6, the "negative control material" indicates that the result was obtained by measuring the cell viability using the negative control material. As a result, it was clarified that the cell viability in all samples was the same as that of the negative control material, and the toxicity with respect to the cells in all films was not recognized.

<<Animal implantation test>>

The biocompatibility of the film was examined by performing the animal implantation test on the film composition similar to that used in the cytotoxicity test. Specifically, the biocompatibility was evaluated by subcutaneously implanting each sample in a rat and observing the surrounding tissues after two weeks and four weeks.
As a result, it was clarified that abnormal inflammation or denaturation was not recognized in all films, and the films have the biocompatibility similar to that of medical stainless steel (SUS316L).

[Experimental Example 5]

(Blood compatibility test)

A blood compatibility test was performed on each film sample. Specifically, the hemolysis (breakdown of red blood cells) and coagulation (blood coagulation due to the foreign matter reaction) were evaluated by bringing each film sample into contact with the human blood.

<<Hemolysis test>>

The hemolysis was examined using the film composition similar to that used in the cytotoxicity test. FIG. 7 is a graph showing the results obtained by measuring the hemolysis rate of the blood which was brought into contact with each sample. In FIG. 7, the "negative control material" indicates the result of a high-density polyethylene film, and the "positive control material" indicates the results of a nonionic surfactant-containing polyvinyl chloride pellet.
As a result, it was clarified that a large difference in hemolysis was not recognized between all films and the negative control material.

<<Blood coagulation test>>

The blood coagulation was examined using the samples prepared in the same manner as in Experimental Example 1. The samples listed in Table 3 were used as the samples. Further, a blood bag (Terumo Corporation: Terumo separation bag) having low blood coagulation was used as the control material. In Table 3, "performed" in the columns of "irradiation with UV" indicates that light having ultraviolet rays including a wavelength of 185 nm was applied, and "not performed" in the columns of "irradiation with UV" indicates that ultraviolet rays were not applied. The "sp$^2$-C (at%)" indicates the proportion (atom%) of the number of carbon atoms having an sp$^2$-hybrid orbital to the total number of carbon atoms having an sp$^2$-hybrid orbital and carbon atoms having an sp$^3$-hybrid orbital in a case of the amorphous carbon and indicates the proportion (atom%) of the number of carbon atoms having an sp$^2$-hybrid orbital to the total number of carbon atoms having an sp$^2$-hybrid orbital, carbon atoms having an sp$^3$-hybrid orbital, and titanium atoms in a case of the titanium-doped amorphous carbon. The "Ti/C (at%)" indicates the proportion (atom%) of the number of titanium atoms to the number of carbon atoms, and the "contact angle" indicates the static contact angle with pure water which was measured according to the liquid droplet method.

[Table 3]

| Samples | Explanation | Irradiation with UV | sp$^2$-C (at%) | Ti/C (at%) | Contact angle (°) |
|---|---|---|---|---|---|
| a | Only substrate | Not performed | - | - | 55.5 |
| b | Amorphous carbon | Not performed | 22 | - | 55.4 |
| c | Amorphous carbon | Not performed | 28 | - | 56.1 |
| d | Amorphous carbon | Not performed | 37 | - | 58.2 |
| e | Amorphous carbon | Not performed | 44 | - | 62.3 |
| f | Titanium-doped amorphous carbon | Not performed | 68 | 2.0 | 60.6 |
| g | Titanium-doped amorphous carbon | Not performed | 75 | 5.3 | 62.4 |
| i | Titanium-doped amorphous carbon | Not performed | 63 | 28.2 | 64.7 |
| k | Amorphous carbon | Performed | 22 | - | 4.4 |
| o | Titanium-doped amorphous carbon | Performed | 68 | 2.0 | 3.5 |

Specifically, first, the human blood was brought into contact with each sample, and the sample was shaken at 37°C for 4 hours. Next, a thrombin-antithrombin complex (TAT) serving as an indicator of the blood coagulation, β-thromboglobulin (β-TG) serving as an indicator of the platelet activity, and C5a serving as an indicator of the inflammatory reaction were measured. Further, the surface of each film was observed using a scanning electron microscope.

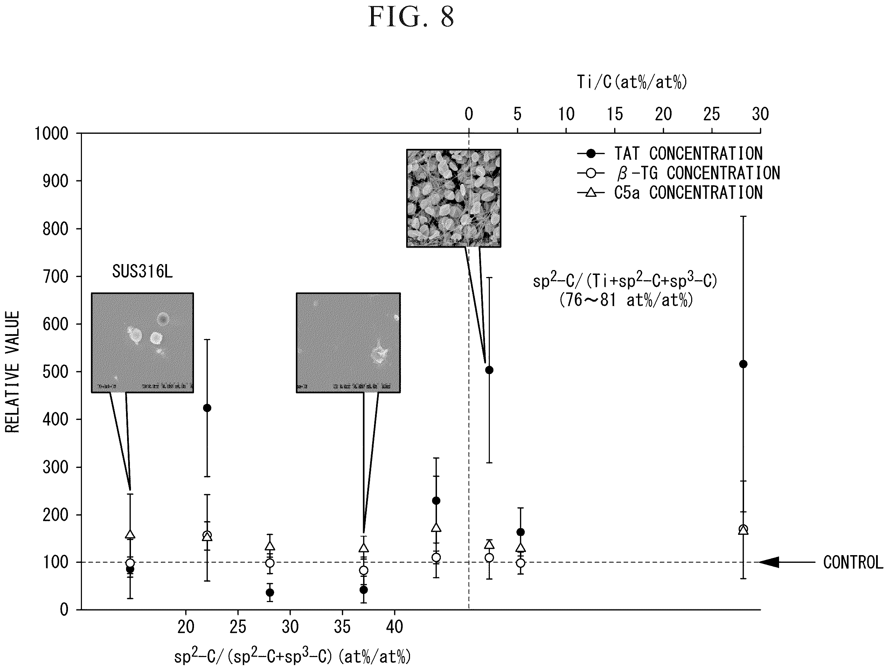

FIG. 8 is a graph showing the results obtained by measuring the concentrations of TAT, β-TG, and C5a. The values in the graph are relative values obtained by setting the measured values in the control material to 100. In FIG. 8, the "control" indicates the result of the control material, the "SUS316L" indicates the sample was formed of only a substrate, and the "at%" indicates atom%. Further, the microphotographs in FIG. 8 are representative scanning electron microphotographs of the film surfaces. Based on the scanning electron microphotographs, it was found that blood cells including a large amount of fibrin chains and red blood cells are observed on the film in a case where blood coagulation occurs, and blood cells are unlikely to be observed in a case where blood coagulation does not occur.

As a result, it was clarified that the concentration of TAT serving as an indicator of blood coagulation was low in the amorphous carbon film in which the proportion of the number of carbon atoms having an sp$^2$-hybrid orbital to the total number of carbon atoms having an sp$^2$-hybrid orbital and carbon atoms having an sp$^3$-hybrid orbital was in a range of 23 to 43 atom% and the titanium-doped amorphous carbon film in which the proportion of the number of titanium atoms to the number of carbon atoms was in a range of 3 to 25 atom%.

Particularly, it was clarified that the amorphous carbon film in which the proportion of the number of carbon atoms having an sp$^2$-hybrid orbital to the total number of carbon atoms having an sp$^2$-hybrid orbital and carbon atoms having an sp$^3$-hybrid orbital was in a range of 23 to 43 atom% exhibited the TAT concentration lower than that of the control material and exhibited the blood compatibility higher than that of the blood bag of the related art serving as the control material.

Although not illustrated in FIG. 8, it was clarified that the blood coagulation was significantly increased in the amorphous carbon film irradiated with ultraviolet rays or the titanium-doped amorphous carbon film irradiated with ultraviolet rays, compared to the blood coagulation in the film which had not been irradiated with ultraviolet rays.

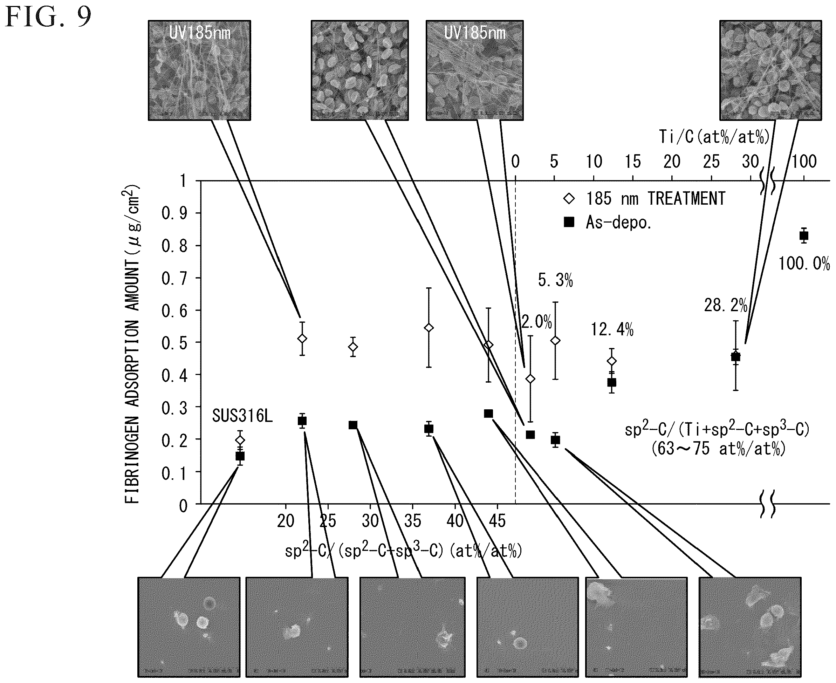

FIG. 9 shows representative electron microphotographs from the blood coagulation test on the graph showing the fibrinogen adsorption amount of each film measured in Experimental Example 2. As a result, it was clarified that the correlation between the protein adsorption properties and the blood coagulation of each film was high. In other words, there was a tendency that a film with excellent protein adsorption properties had high blood coagulation. Based on the results, it was clarified that both the protein adsorption properties and the blood compatibility were excellent in the case of the titanium-doped amorphous carbon in which the proportion of the number of titanium atoms to the number of carbon atoms was in a range of 3 to 12 atom%.

[Experimental Example 6]

(Analysis of outermost film composition)

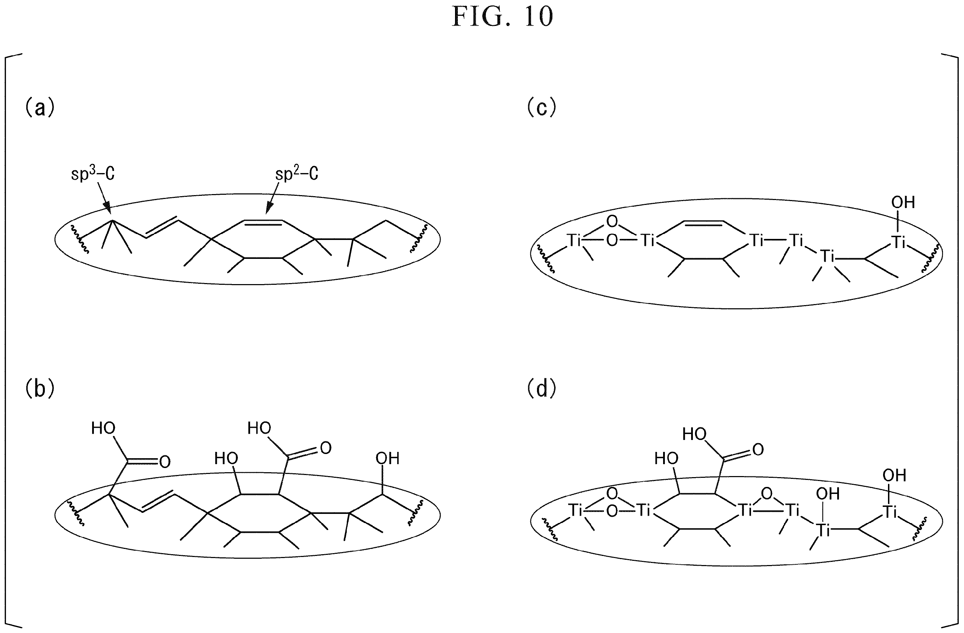

The chemical structure of the outermost surface of each sample prepared in Experimental Example 1 was analyzed based on the time-of-flight secondary ion mass spectrometry (TOF-SIMS) analysis and XPS. FIG. 10 (a) to FIG. 10 (d) are schematic views illustrating a surface chemical structure of each film as determined by TOF-SIMS and XPS analysis.

FIG. 10 (a) shows the surface chemical structure of the amorphous carbon film. In FIG. 10 (a), the "sp$^2$-C" indicates the carbon atoms having an sp$^2$-hybrid orbital, and the "sp$^3$-C" indicates the carbon atoms having an sp$^3$-hybrid orbital. It was clarified that the amorphous carbon film has a surface skeleton formed of carbon atoms having an sp$^2$-hybrid orbital and carbon atoms having an sp$^3$-hybrid orbital.

Further, FIG. 10 (b) shows the surface chemical structure of the amorphous carbon film irradiated with ultraviolet rays. As a result, it was clarified that a hydroxyl group and a carboxyl group are formed.

Further, FIG. 10 (c) shows the surface chemical structure of the titanium-doped amorphous carbon film. It was clarified that TiO or TiOH which can serve as a functional group is present in the titanium-doped amorphous carbon film.

Further, FIG. 10 (d) shows the surface chemical structure of the titanium-doped amorphous carbon film irradiated with ultraviolet rays. As a result, it was clarified that a hydroxyl group or a carboxyl group is formed in the titanium atom or the carbon skeleton, and the amount of TiO or TiOH is increased.

Reference Signs List

100: article
110: base material
120: first film
130: second film
200: FCVA apparatus
201: arc plasma generation chamber
202: raw material target
203: electromagnetic coil
204: ion scan coil
205: spatial filter
206: film deposition chamber
207: substrate holder

**Claims**

1. An article used in contact with a living body or a biological sample, the article comprising:

a first film; and
a second film,
wherein the first film includes an amorphous carbon film in which a proportion of the number of carbon atoms having an sp$^2$-hybrid orbital to a total number of carbon atoms having an sp$^2$-hybrid orbital and carbon atoms having an sp$^3$-hybrid orbital is in a range of 23 to 43 atom% or a titanium-doped amorphous carbon film in which a proportion of the number of titanium atoms to the number of carbon atoms is in a range of 3 to 12 atom%, and the second film includes any one film selected from an amorphous carbon film in which a contact angle with pure water is 10° or less, a titanium-doped amorphous carbon film in which a proportion of the number of titanium atoms to the number of carbon atoms is less than 3 atom% or greater than 12 atom%, or a titanium-doped amorphous carbon film in which a contact angle with pure water is 10° or less.

2. The article according to Claim 1,
wherein a hydroxyl group and/or a carboxyl group is formed in at least a portion of a surface of the amorphous carbon film in which the contact angle with pure water is 10° or less.

3. The article according to Claim 1 or 2,
wherein a hydroxyl group and/or a carboxyl group is formed in at least a portion of a surface of the titanium-doped amorphous carbon film in which the contact angle with pure water is 10° or less.

4. The article according to any one of Claims 1 to 3,
wherein the amorphous carbon film in which the contact angle with pure water is 10° or less and the titanium-doped amorphous carbon film in which the contact angle with pure water is 10° or less are subjected to a treatment of decreasing the contact angle, and
the contact angle with pure water is a numerical value measured immediately after the treatment.

5. The article according to any one of Claims 1 to 4,
   wherein a proportion of the number of carbon atoms having an $sp^2$-hybrid orbital to a total number of carbon atoms having an $sp^2$-hybrid orbital, carbon atoms having an $sp^3$-hybrid orbital, and titanium atoms in the titanium-doped amorphous carbon film is 60 atom% or greater.

6. The article according to any one of Claims 1 to 5,
   wherein the first film has intermediate water having a melting point of -80°C or higher and lower than 0°C.

7. The article according to any one of Claims 1 to 6,
   wherein the first film and the second film each have intermediate water having a melting point of -80°C or higher and lower than 0°C, and
   a mass of the intermediate water contained in the first film is greater than a mass of the intermediate water contained in the second film.

8. The article according to any one of Claims 1 to 7,
   wherein the first film and/or the second film is divided into a plurality of regions.

9. A medical instrument, which is obtained by using the article according to any one of Claims 1 to 8.

10. The medical instrument according to Claim 9,
    wherein the first film is provided in correspondence with a region of the article in contact with the blood.

11. The medical instrument according to Claim 9 or 10,
    wherein the second film is provided in correspondence with a region of the article in contact with a living body.

12. An artificial joint, which is obtained by using the article according to any one of Claims 1 to 8.

13. A medical instrument, which has at least a portion coated with an amorphous carbon film in which a proportion of the number of carbon atoms having an $sp^2$-hybrid orbital to a total number of carbon atoms having an $sp^2$-hybrid orbital and carbon atoms having an $sp^3$-hybrid orbital is in a range of 23 to 43 atom% or a titanium-doped amorphous carbon film in which a proportion of the number of titanium atoms to the number of carbon atoms is in a range of 3 to 12 atom%.

14. A medical instrument, which has at least a portion coated with any one film selected from an amorphous carbon film in which a contact angle with pure water is 10° or less, a titanium-doped amorphous carbon film in which a proportion of the number of titanium atoms to the number of carbon atoms is less than 3 atom% or greater than 12 atom%, and a titanium-doped amorphous carbon film in which a contact angle with pure water is 10° or less.

15. The medical instrument according to Claim 14,
    wherein the amorphous carbon film in which the contact angle with pure water is 10° or less and the titanium-doped amorphous carbon film in which the contact angle with pure water is 10° or less are subjected to a treatment of decreasing the contact angle, and
    the contact angle with pure water is a numerical value measured immediately after the treatment.

16. The medical instrument according to Claim 14 or 15,
    wherein a proportion of the number of carbon atoms having an $sp^2$-hybrid orbital to a total number of carbon atoms having an $sp^2$-hybrid orbital, carbon atoms having an $sp^3$-hybrid orbital, and titanium atoms in the titanium-doped amorphous carbon film is 60 atom% or greater.

# FIG. 1

# FIG. 2

○ NEUTRAL PARTICLE
● + ION

FIG. 3

FIG. 4

EP 3 613 445 A1

FIG. 5

EP 3 613 445 A1

## FIG. 6

⊠ NEGATIVE CONTROL MATERIAL
□ SAMPLE

## FIG. 7

# FIG. 8

FIG. 9

FIBRINOGEN ADSORPTION AMOUNT ($\mu$ g/cm$^2$)

Ti/C (at%/at%)

185 nm TREATMENT
◇ 185 nm As-depo.
■ As-depo.

100.0%
28.2%
12.4%
5.3%
2.0%

sp$^2$-C/(Ti+sp$^2$-C+sp$^3$-C)
(63~75 at%/at%)

sp$^2$-C/(sp$^2$-C+sp$^3$-C) (at%/at%)

SUS316L

UV185nm
UV185nm

FIG. 10

(a)

(b)

(c)

(d)

EP 3 613 445 A1

FIG. 11

(a)

(b)

# FIG. 12

(a)

(b)

# FIG. 13

(a)

120, 130

100

110

(b)

120, 130

100

110

120, 130

**EP 3 613 445 A1**

## INTERNATIONAL SEARCH REPORT

| International application No. |
|---|
| PCT/JP2018/011019 |

### A. CLASSIFICATION OF SUBJECT MATTER

Int.Cl. A61L27/30(2006.01)i, A61F2/30(2006.01)i, A61L15/18(2006.01)i, A61L17/00(2006.01)i, A61L17/14(2006.01)i, A61L27/40(2006.01)i, A61L29/10(2006.01)i, A61L29/12(2006.01)i, A61L31/08(2006.01)i, A61L31/12(2006.01)i, A61L33/02(2006.01)i, C12M1/00(2006.01)i

According to International Patent Classification (IPC) or to both national classification and IPC

### B. FIELDS SEARCHED

Minimum documentation searched (classification system followed by classification symbols)

Int.Cl. A61F2/00, 2/02-2/80, 3/00-4/00, A61L15/00-33/18, B01L1/00-99/00, C12M1/00-3/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

JSTPlus/JMEDPlus/JST7580(JDreamIII), Google

### C. DOCUMENTS CONSIDERED TO BE RELEVANT

| Category* | Citation of document, with indication, where appropriate, of the relevant passages | Relevant to claim No. |
|---|---|---|
| Y<br>A | JP 2005-320585 A (NIPPON ITF, INC.) 17 November 2005, claims 1, 6, paragraphs [0010], [0021], [0025], [0030], [0038], [0047], [0061]<br>(Family: none) | 1-10, 13-16<br>11, 12 |
| Y<br>A | JP 2008-230880 A (TOYO ADVANCED TECHNOLOGIES CORPORATION) 02 October 2008, claims 1, 2<br>(Family: none) | 1-10, 13-16<br>11, 12 |
| Y | 中東孝浩ら, ゴム・高分子材料向け DLC (フレキシブル DLC), 精密工学会誌, 2002, 68(12), pp. 1530-1534, particularly, p. 1533, column 7.7, (NAKAHIGASHI, Takahiro et al., DLC(flexible DLC), Journal of the Japan Society of Precision Engineering) | 8-10 |

☒ Further documents are listed in the continuation of Box C.　　　☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 12.04.2018 | 01.05.2018 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2018/011019

C (Continuation).    DOCUMENTS CONSIDERED TO BE RELEVANT

| Category* | Citation of document, with indication, where appropriate, of the relevant passages | Relevant to claim No. |
|---|---|---|
| A | JP 11-318960 A (NISSIN ELECTRIC CO., LTD.) 24 November 1999, entire text, all drawings (Family: none) | 1-16 |
| A | WO 2005/011765 A1 (IWAMOTO, Yukihide) 10 February 2005, page 7, lines 1, 2 & US 2008/0039939 A1 & EP 1649875 A1 | 11, 12 |

Form PCT/ISA/210 (continuation of second sheet) (January 2015)

**REFERENCES CITED IN THE DESCRIPTION**

**Patent documents cited in the description**

- JP 2017083711 A
- JP 2017128332 A
- JP 2014104175 A